# EUROPEAN PATENT SPECIFICATION

(11) **EP 2 565 453 B1**
(45) Date of publication and mention of the grant of the patent: **16.11.2016**
(21) Application number: 12180276.3
(22) Date of filing: 13.08.2012
(51) Int. Cl.: F04B 43/12, A61M 5/142, A61M 1/10

(54) **Tube Accommodation of a Peristaltic Roller Pump**
Schlauchaufnahme einer peristaltischen Rollenpumpe
Logement de tube d'une pompe péristaltique à rouleaux

(30) Priority: 29.08.2011 DE 102011081722
(43) Date of publication of application: 06.03.2013
(73) Proprietor: AESCULAP AG, 78532 Tuttlingen (DE)
(72) Inventor: Schönewerk, Erik, 78166 Donaueschingen (DE); Schnekenburger, Rolf, 78532 Tuttlingen (DE); Schneider, Jürgen, 78532 Tuttlingen (DE)
(74) Representative: Winter, Brandl, Fürniss, Hübner, Röss, Kaiser, Polte - Partnerschaft mbB

(56) References cited:
- EP-A1- 1 518 572
- WO-A1-02/48549
- US-A- 3 675 653
- US-A- 4 256 442
- US-A- 5 928 177
- US-A1- 2005 254 978

## Description

The present invention relates to a peristaltic pump and in particular to a tensioning device arranged in/at a tube accommodation chamber of the peristaltic or roller pump for impacting tension to a tube adapted to be inserted in the duct, preferably of disposable construction.

Roller or peristaltic (squeezing) pumps are displacement pumps in which the medium to be pumped is pressed through a tube by a flexible outer mechanical deformation thereof. Due to the gentle advancement of the medium (without it being sheared or distorted), peristaltic pumps are especially also used for medical purposes.

In the casing of a generic peristaltic pump a flexibly/elastically deformable tube is on principle inserted such that it is guided around a rotatable pump head along a predetermined circumferential section on which a number of rollers spaced apart at angles are rotatably mounted, said rollers being, with an appropriate rotation of the pump head, sequentially brought into rolling engagement with the tube and compress/clamp same. The tube either supports itself at the outer surface of the pump casing or is tensioned around and against the pump head with a predetermined tensile force.

For example, peristaltic pumps in which the tube supports itself at a surface of a pump casing or the like, are known from EP 1 518 572 A1, US 5 928 177 A, US 3 675 653 A, US 2005/2549978 A1, US 4 256 442 A or WO 02/48549 A1. Further, from US 5 443 451, a peristaltic pump comprising a holder for holding a flexible tube at two axially spaced-apart positions is known.

With any construction, however, the movement of the pump head results in that the respective clamping position of a roller that is being in engagement moves forward along the tube and thus advances the pumping medium inside the tube section immediately in front of the corresponding roller. The generation of a suction underpressure in the tube section immediately following the corresponding roller is, as a rule, effected by the inherent elasticity of the tube material due to which the tube attempts to recover its original shape of construction.

Due to increasingly stricter hygienic provisions and due to more demanding requests of users it has become usual to design at least those components of a peristaltic pump that get into direct contact with the pumping medium as a disposable, in particular when used in medicine,. This includes primarily the tube which consequently has to be adapted to be removed and inserted easily. In prior art, a plurality of tube units and tensioning devices are known, some of which are indicated in the following.

Basically, there exists the possibility of preassembling the tube and the pump head in a kind of cartridge as a separate pump unit that, in the case of a changing process, merely has to be inserted/latched into the casing of a peristaltic pump such that the pump head is engaged in a rotation-fixed manner with a drive shaft, for instance, of an electric motor or a gear mechanism downstream thereof. Although this solution greatly facilitates the changing process and simultaneously also ensures high operating safety since it is possible to design the cartridge as a self-contained casing unit, so that rotating elements are housed to be inaccessible from outside, this is a relatively expensive variant of construction.

The pump head is a rather complex component consisting of a plurality of individual parts movable/rotating relative to each other (as was already indicated before), which in fact would not have to be changed since it is not contaminated directly by the pumping medium. It is therefore substantially more cost-efficient to provide only the tube as a disposable. For changing the tube, however, it has to be drawn off the pump head or fitted on it again, respectively, wherein a pre-tensioning force by which the tube is tensioned around the pump head has to be overcome. To facilitate the changing of merely the tube (with the pump head remaining), a specific tensioning mechanism has therefore been developed by means of which it is possible to release and tension the tube for a changing process.

G 94 11 183 discloses a simple construction of such tensioning device. Accordingly, the tube is provided/designed with two axially spaced-apart bulge-shaped stops/holders, whereas the pump casing is, inside the tube accommodation chamber molded or inserted therein, provided with a transverse strip or a beam as a tube accommodation in which two indentations are formed. In order to insert the tube in the tube accommodation, one of the bulge-shaped stops is latched/hooked into one of the indentations and subsequently the tube is pulled around the pump head. Finally, the other bulge-shaped stop is latched/hooked into the other indentation. This insertion process is very time-consuming and complicated since the stops have to be hooked into the indentations while the tube is under tension, which requires some skill and force by an operator.

Alternatively, it is also possible to provide a catching beam as a tube accommodation which is, at the two axial ends thereof, fixedly connected with the tube at two axially spaced-apart tube sections such that the tube (tube middle section) forms a loop between the beam ends. The beam and the tube thus form a separate, exchangeable tube unit.

The tube formed this way is now placed/guided with its loop area around the pump head, and the catching beam is latched into a tensioning device. The tensioning device preferably provides a guide rail/guide groove for the catching beam along which the catching beam is displaced while the loop area is tensioned until the catching beam latches into a detachable holder/latch in the tube accommodation chamber. Although this construction facilitates the inserting process of the tube, the tube has to be tensioned in the region of the loop simultaneously at two ends of the tube area and hence at two edges forming with respect to the pump head, which requires stronger force that is not possible to be exerted by every operator.

Finally, the changing of merely the tube (and not the pump head) necessitates free access from outside of the rotating components, too, said access remaining open also during operation for cost reasons or being at least partially closable by means of an additional service/protection cap. The latter does not only increase the price of the peristaltic pump, but also constitutes a safety hazard since the cap has to be closed in an additional step, which may be forgotten.

In view of these problems it is an object of the present invention to provide a peristaltic pump of the instant type which has increased functionality. It is an object to facilitate the inserting process for a tube or a tube unit especially of disposable construction. Another object is to improve the safety of the peristaltic pump. Finally, it is an object of the invention keep the peristaltic pump and in particular the exchange tube unit cost-efficient.

The foregoing object is solved by a peristaltic pump with the features of claim 1 and by a tube unit pursuant to claim 10. Advantageous developments of the invention are the subject matters at least of the subclaims.

A basic idea of the invention accordingly consists in providing the peristaltic pump with a tube accommodation chamber in which the tube may be inserted while it is almost untensioned. Furthermore, the peristaltic pump comprises a tube tensioning device with a lever mechanism adapted to be operated preferably by hand or by electric motor so as to tension the tube along a predetermined circumferential section around the rotating pump head of the peristaltic pump. The lever mechanism enables the operating force required for reaching the necessary tensioning force and hence the pressure force against the pump head to be reduced, so that is can simply be exerted by hand without additional separate tools being required. In the case of a motor operation of the lever mechanism, the drive may be of small dimension and hence cost-efficient.

Basically, a plurality of constructional designs are conceivable as a lever mechanism, by mean of which an operating movement is transferrable with an appropriate reduction to the tube / the tube unit, such as, for instance, a worm gear, a lifting block, a gear mechanism, or similar auxiliary means. Practically it is, however, advantageous to design the lever mechanism, in particular in the case of manual operation, conventionally in the form of an operating element (operating handle) hingedly mounted to the peristaltic pump and coupled (operatively connected) with a displaceable component or plunger component transferring thrust, which transfers an operating force generated at the operating handle by pivoting as a function of the kind/place of coupling in a reduced manner to the tube. Such a concept for the lever mechanism is easy to manufacture, fail-safe and hence economic.

In accordance with one aspect of the invention there is consequently provided a medical peristaltic pump comprising a pump casing in which a tube accommodation chamber is formed or accommodated in which a pump head is rotatably mounted, and a tube unit inserted in the tube accommodation chamber such that a tube of the tube unit is engaged or is adapted to be engaged with the pump head in a manner to encompass same at least partially.

The tensioning force with which the tube touches or is to touch the pump head is exerted by a tensioning device arranged at the pump casing (i.e. provided as a fixed component of the peristaltic pump), said tensioning device cooperating with the tube unit in order that during its (manual/motor) operation the tube is tensioned around the pump head. In accordance with the invention, the tensioning device is a lever mechanism fixedly mounted to the pump casing and hence not to be considered as a tool.

Due to this design it is easy for any person to insert and tension a tube or a tube unit. The removal of a tube or a tube unit is also easy to perform in that the pre-tension of the tube around the pump head is released by the lever mechanism and subsequently the tube/tube unit is removed from the tube accommodation chamber almost free of force. Since the lever mechanism is a component of the peristaltic pump, it is not changed each time, so that the operation of the peristaltic pump remains cost-efficient.

Preferably, a lever element of the lever mechanism simultaneously also comprises a protective cap for at least partially covering the tube accommodation chamber. Thus, on operating the tensioning device, the tube accommodation chamber is simultaneously opened and/or closed (automatically), and hence the movable parts positioned therein, such as the pump head, are protected toward the outside. This increases the safety of the peristaltic pump.

Another aspect of the invention provides, where appropriate, that the protective cap either forms or is fixed to that lever element that is provided as an operating handle of the lever mechanism, or forms or is fixed to that lever element (plunger element) that is adapted to be operated via the operating handle for the transfer of forces. As was already explained at the beginning, the lever mechanism according to the invention comprises an operating element or an operating handle that is directly or indirectly hingedly mounted to the pump casing, and a plunger element that is operatively connected with the lever for a transfer of forces. The protective cap may accordingly be assigned either to the operating handle or to the plunger element.

In case the operating handle simultaneously comprises or is the protective cap, it may have the shape of a cover plate. In case the force transferring lever element (plunger element) comprises or is the protective cap, the operating handle may have the shape of a bow-shaped handle, a buckle/operating button, a toggle lever, or a similar ergonomic shape. Since the protective function and the operating function are accordingly assigned to different elements of the lever mechanism, it is possible to adapt their constructions optimally to their remaining functions without any compromise.

In accordance with another aspect of the invention there may be provided a tube accommodation (hook component) that is arranged or adapted to be arranged either fixedly or displaceable in or at the tube accommodation chamber, and into which the tube is adapted to be inserted or is inserted/hooked via a tube holder formed or fixed thereon. It is of advantage if, in the case of a fixed tube accommodation, the lever mechanism acts on the now displaceable pump head to press it against a free tube middle section positioning itself in the fixed tube accommodation. In the case of a displaceable tube accommodation there is provided in an advantageous manner that the lever mechanism acts on the tube accommodation preferably as a further element of the lever mechanism to move it relative to the now stationary (not displaceable) pump head such that a free tube middle section positioning itself in the tube accommodation touches the pump head in a manner encompassing same. In the inserted, untensioned state the tube middle section may be straight or may already be looped.

It may furthermore be of advantage if the lever mechanism is designed to be self-locking, i.e. if the operating handle, when operated, swivels across a kind of dead center for tensioning the tube and then folds/snaps quasi automatically to its final tensioning position. This final tensioning position may be supported by a slight lock-in position for tactile feedback. This makes an additional safety element such as a locking bolt superfluous, so that operation is facilitated and the safety of the peristaltic pump is further increased.

Finally, in accordance with another aspect of the invention there may be provided to provide a tube unit for a peristaltic pump in accordance with the invention (separately of the peristaltic pump according to the invention), consisting of a tube holder (engagement device) holding the flexible, preferably elastic tube at two axially spaced-apart positions, or being fixedly connected with the tube at these positions, respectively, and thus forming at least one undercut adapted to be brought in holding engagement with the tube accommodation of the peristaltic pump or the lever mechanism. Thus, it is possible to manufacture the tube unit in a very cost-efficient manner due to the few components thereof, and it is therefore particularly suited as a disposable.

In accordance with another aspect of the present invention there is provided a medical peristaltic pump comprising
- a pump casing in which a tube accommodation chamber is formed or inserted in which a pump head is rotatably mounted; and
- a tube unit provided preferably as an exchange component and consisting of a tube holder adapted to be inserted displaceably in the tube accommodation chamber and holding a flexible tube in the form of a loop at two axially spaced-apart tube positions, wherein the tube unit is adapted to be inserted or is inserted in the tube accommodation chamber such that by the displacement of the tube holder is tensionally engaged with the pump head in a manner to encompass same at least partially.
- In accordance with the invention, there is provided a tensioning device in the form of a lever mechanism which is fixedly mounted (that means not provided for exchange) at the pump casing or at the tube accommodation chamber, said lever mechanism comprising at least one lever element (preferably the operating element) as a (front side) protective cap for at least partially (at the front side) covering/closing the tube accommodation chamber, and cooperating with the tube holder for the displacement thereof so as to pre-tension the tube around the pump head when operated.

By the tensioning of the tube around the pump head it is thus simultaneously covered/closed at least in sections toward the outside (toward the open front side of the tube accommodation chamber) and hence protected from danger.

In the following, the invention will be explained in detail by means of preferred embodiments with reference to the accompanying Figures.
- Fig. 1: shows a first preferred embodiment of the invention with a manually operable lever mechanism as a tensioning device with a stationary tube accommodation and a bow-shaped operating handle in the open (untensioned) and closed (tensioned) conditions,
- Fig. 2: shows a second preferred embodiment of the invention with a manually operable lever mechanism as a tensioning device with a stationary tube accommodation and a buckle-shaped operating handle in the open (untensioned) and closed (tensioned) conditions,
- Fig. 3: shows a third preferred embodiment of the invention with a manually operable lever mechanism as a tensioning device with a stationary tube accommodation and a plate-shaped operating handle in the open (untensioned) and closed (tensioned) conditions,
- Fig. 4: shows a fourth preferred embodiment of the invention with a manually operable lever mechanism as a tensioning device with a stationary tube accommodation and a toggle lever-shaped operating handle in the open (untensioned) and closed (tensioned) conditions,
- Fig. 5: shows a fifth preferred embodiment of the invention with a manually operable lever mechanism as a tensioning device with a displaceable/movable tube accommodation and a button-shaped operating handle in the open and closed conditions,
- Fig. 6: shows a sixth preferred embodiment of the invention with a manually operable lever mechanism as a tensioning device with a displaceable/movable tube accommodation and a plate-shaped operating handle in the open (untensioned) and closed (tensioned) conditions,
- Fig. 7: shows a seventh preferred embodiment of the invention with a lever mechanism operable by an electric motor as a tensioning device with a displaceable/movable tube accommodation and a gear mechanism as a lever mechanism between the drive motor and the tube accommodation in the open (untensioned) and closed (tensioned) conditions, and
- Fig. 8: shows, as an illustrative example, the casing of a peristaltic pump according to the invention with a bow-shaped operating handle pursuant to Fig. 1 and in particular the tube accommodation chamber in the open (untensioned) condition.

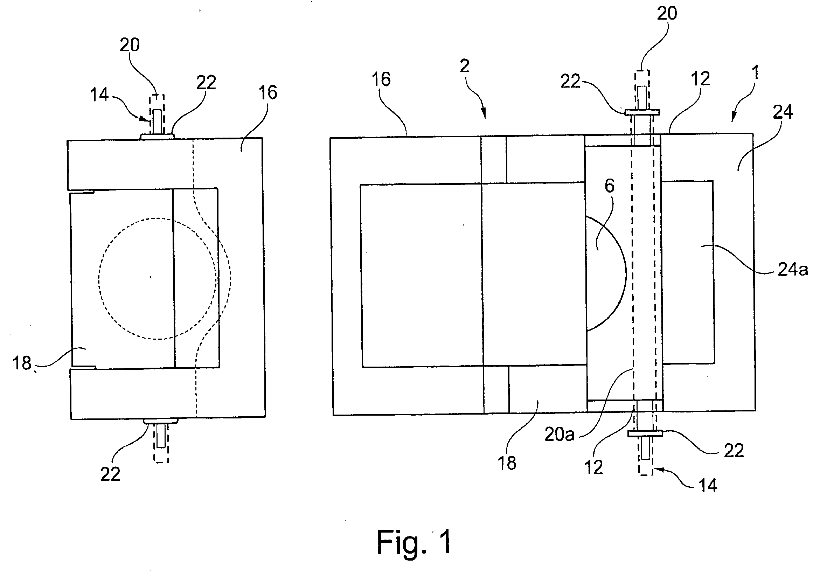

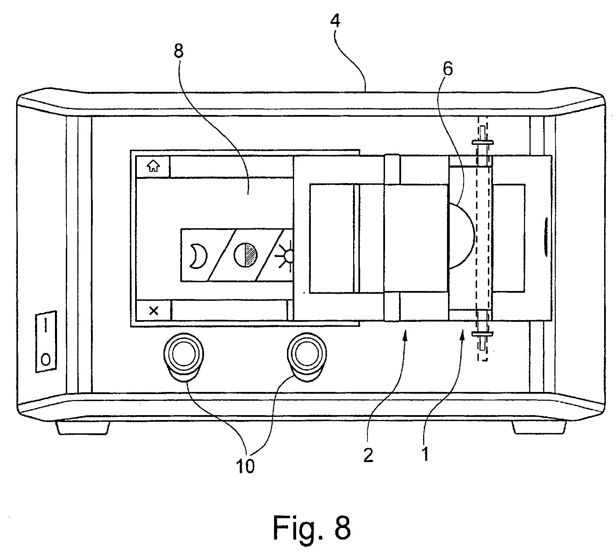

Fig. 1 shows in detail a tube accommodation chamber 1 of a peristaltic pump pursuant to Fig. 8, comprising a tensioning device with a lever mechanism 2. As may be gathered from Fig. 8, the peristaltic pump of the invention basically comprises a pump casing 4 at the front side of which the tube accommodation chamber 1 is arranged such that it opens toward the front side of the pump casing 4. Inside the tube accommodation chamber 1 there is positioned a pump head 6 consisting of a motor-driven, rotatable drive wheel at the radial outer circumference of which a number of pressure wheels/rollers (not illustrated) are mounted to rotate relatively and are distributed at regular circumferential intervals to each other. A control panel 8 that is also placed at the front side of the pump casing 4 serves to program a control at least for the electric drive of the pump head so as to set, for instance, the torque or the tum-on and turn-off times of the peristaltic pump. Moreover, turn-on and turn-off buttons are arranged at the front side of the pump casing.

In the embodiment of Fig. 1, the tube accommodation chamber 1 is designed in the form of a substantially rectangular shell or depression that is either manufactured integrally with the pump casing 4 or inserted as a separate component in a corresponding section of the pump casing 4. At two opposing walls of the tube accommodation chamber 1 respective indentations 12 are formed which serve as tube accommodations and in which a tube or a tube unit 14 may be inserted. The indentations 12 are flush with each other such that their alignment is arranged to be radially displaced to the pump head 6.

At a side of the tube accommodation chamber 1 extending in parallel to the alignment, the tensioning device with the lever mechanism 2 is mounted on the pump casing 4. This lever mechanism consists in the instant case of an operating handle 16 in the form of a grip that is hingedly mounted on the pump casing 4 and that is operatively connected with a shifting element (plunger component) 18 via a (not illustrated) joint, spring means, or a gear mechanism. The shifting element 18 has, in the plan view pursuant to Fig. 1, a substantially rectangular plate shape with a dimension that suffices to cover the tube accommodation chamber 1 largely or even completely. The shifting element 18 is further connected with the axis of rotation of the pump head 6 and forms in the instant case a bearing for the axis of rotation.

The shifting element 18 may, for instance, consist of two plates that are spaced apart in parallel, that are fixed to each other, and that hold the pump head between them in a rotatable manner. In this case it is also of advantage if the (not illustrated) drive motor for the pump head 6 is also fastened to the shifting element 18. The shifting element 18 is guided to be displaced at the pump casing 4 such that it cannot be lifted off the pump casing 4. The mounting of the pump head 6 is further chosen such that the pump head 6 projects over the shifting element 18 at the plate edge of the shifting element 18 which faces the alignment.

Fig. 1 also illustrates the tube unit 14 that is, in accordance with the invention, provided as a component separate of the peristaltic pump and can optionally be inserted in the tube accommodation chamber 1.

The tube unit 14 accordingly consists of a flexible, preferably elastically deformable tube 20 at which two holders or engaging elements 22 are fastened or designed at axial distance to each other. These holders 22 form radial circumference projections or collars that are adapted to be brought into undercut engagement with the indentations 12 at the tube accommodation chamber 1. The axial distance of the holders 22 is chosen such that, if the tube 20 is inserted in the two indentations 12, so that the holders 22 touch the tube accommodation chamber 1 behind the indentations 12, the tube middle section 20a extending therebetween is not or just slightly tensioned, so that it substantially follows the alignment.

At the opposite side of the alignment with respect to the shifting element 18, the tube accommodation chamber 1 is partially covered with a fixed plate 24, wherein the fixed plate 24 comprises an inner bulge 24a into which that part of the pump head 6 that projects from the plate-shaped shifting element 18 in the direction of the alignment immerses or moves in, respectively, when the tube accommodation chamber 1 is closed..

The functioning of the peristaltic pump according to the first preferred embodiment of the invention can be described by means of Fig. 1 and Fig. 8 as follows.

In accordance with Fig. 1, right illustration, the tube accommodation chamber 1 is opened in that the bow-shaped operating handle 16 according to Fig. 1 is folded manually to the left, so that the plate-shaped shifting element 18 along with the pump head 6 is displaced via the lever mechanism to the left, i.e. away from the alignment. In this (open, untensioned) position it is now possible to insert the tube unit 14 in the indentations in accordance with the foregoing description without the exertion of force and hence easily.

Subsequently, the bow-shaped operating handle 16 is folded manually in the direction of the alignment, wherein this folding or swiveling movement is transferred via the lever mechanism with an appropriate reduction to the plate-shaped shifting element 18 for the displacement thereof. That means, by swiveling the operating handle 16 in the direction of the alignment, the plate-shaped shifting element 18 is pushed forward in the direction of the fixed plate 24, wherein the pump head 6 that is rotatably mounted thereon is simultaneously pressed against the tube middle section 20a and pushed forward. Since the movement distance of the shifting element 18 until it abuts on the fixed plate 24 is larger than the distance between the inserted tube 20 and the pump head 6 in the completely open condition, the pump head 6 is consequently quasi pressed through the tube 20, wherein the tube middle section 20a is tensioned between the holders 22 / indentations 12 around the pump head 6 along a partial circumference thereof. In the completely closed condition of the tube accommodation chamber 1 in accordance with the left illustration in Fig. 1, the tube middle section 20a is accordingly elastically deformed in U-shape, so that a predetermined touching force results between the pump head 6 and the tube 20.

In the process of the swiveling movement of the operating handle 16 in the closing or tensioning direction, respectively, it exceeds a kind of dead center of the lever mechanism, whereupon the operating handle 16, utilizing the tensioning force of the tube middle section 20a which has already been generated, is urged on the pump head 6 in the direction of the tensioned final position of the operating handle (pursuant to the left illustration in Fig. 1). That means, the lever mechanism according to the first embodiment of the invention is equipped with a kind of self-locking that keeps the operating handling 16 automatically, without additional locking elements, in the tensioned final position.

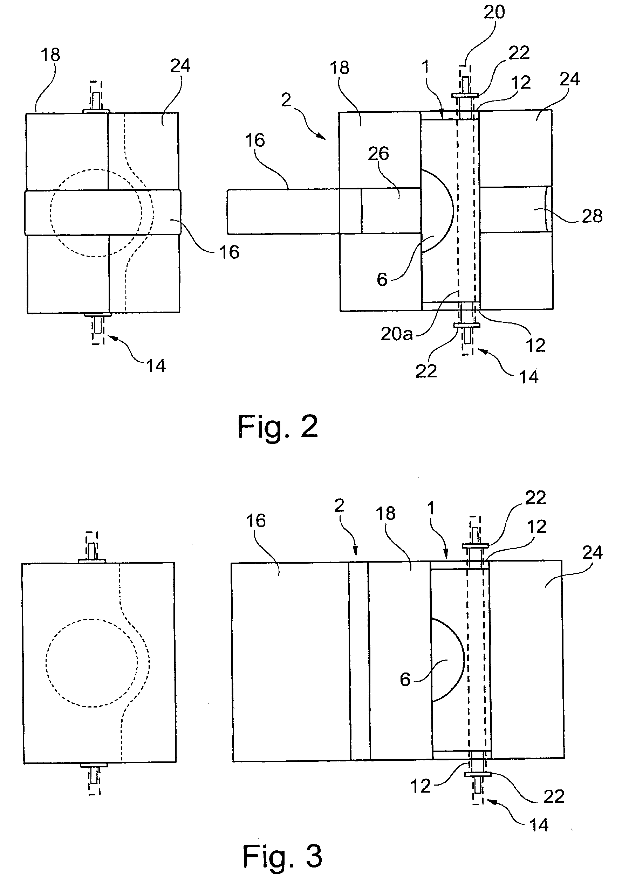

Figs. 2 and 3 illustrate alternative embodiments of the tensioning device 2 pursuant to Fig. 1, wherein merely the technical differences as compared to the first embodiment are to be dealt with in the following while all the other technical features substantially correspond to the first embodiment.

The embodiments pursuant to Figs. 2 and 3 differ from the first embodiment pursuant to Fig. 1 in the design of the manually operable operating handle 16. In accordance with Fig. 2, the operating handle 16 is designed as a strip-shaped buckle whereas the plate-shaped shifting element 18 pursuant to the right illustration in Fig. 2 is designed at its upper side with a longitudinal groove 26 in shifting direction of the shifting element 18 which is facing the buckle 16. In this case, the fixed plate 24 is also provided with a corresponding longitudinal groove 28 that is flush with the longitudinal groove 26 in the shifting element 18.

The longitudinal groove(s) 24, 26 is (are) dimensioned such that the buckle 16 during folding in the tensioned final position pursuant to the left illustration in Fig. 2 swivels into the groove(s) 24, 26 and is accommodated therein almost completely. Thus, a substantially smooth, edge-free upper side of the plate-shaped shifting element 18 and of the fixed plate 24 results, so that an inadvertent opening of the buckle 16, for instance, due to getting caught, is safely avoided. This contributes to the further improvement of the safety of the peristaltic pump according to the invention.

Finally, the operating handle 16 pursuant to the right illustration in Fig. 3 may also consist of a plate-shaped flap that covers, after the folding in the tensioned final position (see left illustration in Fig. 3), the tube accommodation chamber 1, preferably along with the fixed plate 24, completely.

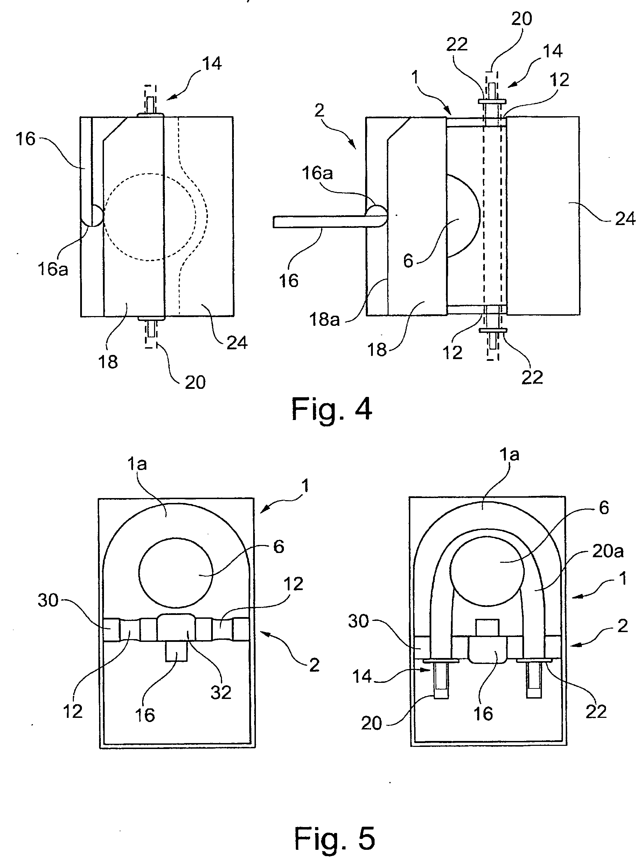

The embodiment according to Fig. 4 basically corresponds to the afore-described embodiments, but in this case the operating handle 16 is designed in the form of a toggle lever. In practice, the toggle lever 16 is hinged at the tube accommodation chamber 1 or at the pump casing 4 such that its swivel axis extends perpendicular to the front side of the pump casing 4 and hence perpendicular to the upper side of the plate-shaped shifting element 18. The toggle lever 16 is designed with an eccentric or a cam 16a pressing against the thrust element 18 on rotation of the lever 16 and displacing same in closing direction against the inserted tube 20. Since the toggle lever 16 is not coupled with the shifting element 18, but only touches one back edge 18a thereof, a spring (means) that is not illustrated is provided in the instant case, said spring (means) pre-tensioning the shifting element 18 in opening direction (untensioned final position pursuant to the right illustration in Fig. 4). The toggle lever 16 is also positioned such that on reaching its tensioned final position (see left illustration in Fig. 4) it has exceeded an (upper) dead center of the cam 16a and is thus kept automatically in this position.

All embodiments pursuant to Figs. 1 to 4 have in common that the tube accommodation in the form of the two flush indentations 12 is formed/arranged stationary in the tube accommodation chamber/duct 1, so that it is necessary for a tensioning of the tube 20 to mount the pump head 6 to be displaceable in the direction of the tube unit 14. There is, however, also basically the possibility of holding the pump head 6 stationary in the tube accommodation chamber 1 and of mounting/guiding instead the tube accommodation 12 to be displaceable in the tube accommodation chamber 1. In this case the tensioning device 2 need not act on the pump head 6, but on the tube accommodation 12. In the following, this functional principle will be exemplified by means of Figs. 5 to 7.

Fig. 5 accordingly shows a fifth embodiment of the invention as a simple construction for implementing the afore-mentioned alternative tensioning principle.

In this case, the pump head 6 is, pursuant to Fig. 5, left illustration, rotatably mounted (stationary) in the tube accommodation chamber 1 and is driven by a motor that is not illustrated in the pump casing 4. The tube accommodation chamber 1 is guided in a semi-circle around the pump head 6, so that a kind of U-shaped accommodation duct or groove 1 a is formed between the tube accommodation chamber 1 and the pump head 6.

At the side of the pump head 6 radially opposite to the accommodation duct 1 a, a beam-shaped accommodation 30 is mounted in the tube accommodation chamber 1 such that the beam 30 that is oriented substantially tangentially to the pump head 6 is displaceable in the transverse direction thereof. In a middle section of the beam 30 (the beam-shaped accommodation), a longitudinal slit 32 extending in the displacement direction of the beam 30 is formed in the tube accommodation chamber 1, through which a lever-shaped operating handle 16 extends which has the shape of a button at its free projecting end. At its other, invisible end the operating handle 16 is pivotally mounted in the pump casing 4. In an axial middle section the lever-shaped operating handle 16 comprises a catching element that is not illustrated and that is in operative engagement with the beam-shaped accommodation 30 so as to transform a swiveling movement of the operating handle 16 with an appropriate reduction of the lever mechanism generated thereby to a displacing movement of the beam 30.

As may further be taken from Fig. 5, right illustration, the tube unit 14 also comprises two axially spaced-apart collar-shaped holders 22 that are adapted to be brought into undercut engagement with indentations 12 in the displaceable beam-shaped accommodation 30. In this case, however, the axial distance between the two holders 22 along the tube 20 is so large that the tube middle section 20a bulges to a U-shaped loop between the holders 22 when the holders 22 or the tube 20, respectively, are/is placed/inserted in the beam-shaped tube accommodation 30 at the indentations 12 thereof. The loop has a middle radius corresponding approximately to the U-shaped accommodation duct 1 a around the pump head 6, so that the tube middle section 20a may be placed around the pump head 6 in the untensioned condition almost without any effort.

As soon as the tube unit 14 has been inserted in the tube accommodation duct 1 a, the operating handle 16 is operated, i.e. in the instant case swiveled away from the pump head 6, and thus the beam-shaped accommodation 30 is moved away from the pump head 6. Thus, the tube middle section 20a touches the partial circumference of the pump head 6 with a predefined tensioning force, as is illustrated in the right illustration in Fig. 5.

As in the afore-described embodiments, the operating handle 16 pursuant to the fifth preferred embodiment of the invention is dimensioned such that it exceeds, shortly prior to reaching its tensioned final position (see right illustration in Fig. 5), a dead center whereupon the tensioning force of the tube 20 holds the operating handle 16 in this tensioned final position.

The fifth embodiment of the invention pursuant to Fig. 5 has a relatively simple mechanical construction since the pump head 6 can be kept stationary and merely the (preferably beam-shaped) accommodation 30 has to be displaceable relative to the pump head 6. Thus, this construction can be manufactured in a cost-efficient manner. However, with this solution the rotating parts are exposed even in the tensioned final position of the operating handle 16.

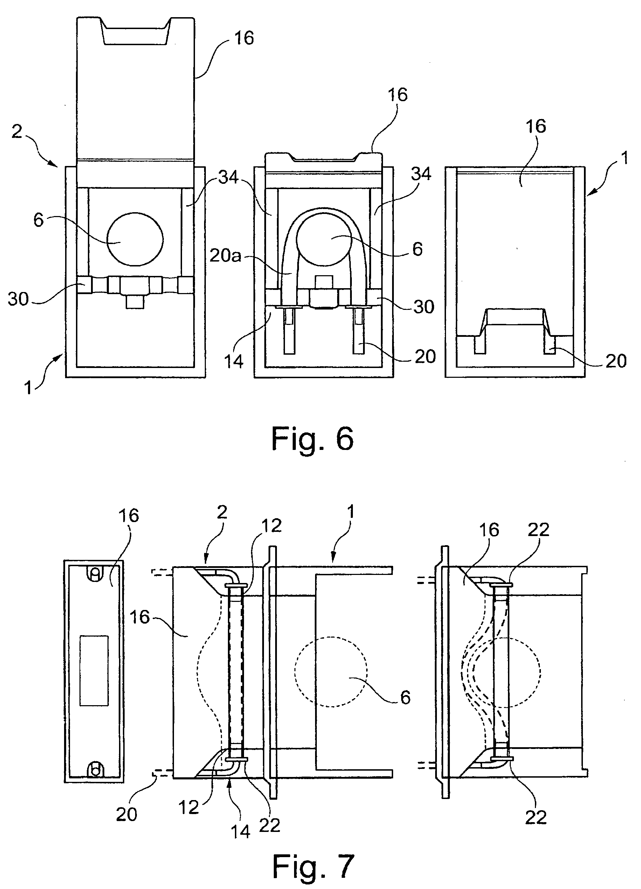

For solution of this problem the sixth embodiment of the invention pursuant to the left illustration in Fig. 6 provides a modified operating handle 16 as compared to the fifth preferred embodiment. In this case, the afore-described button is replaced by a plate-shaped flap that is hinged to the pump housing 4 by means of a hinge that is not illustrated, and that is operatively connected with the beam-shaped accommodation 30 via a kind of plunger component 34. The plunger component may in the instant case consist of a bar frame that is hinged at a particular middle position to the pivotable flap 16 and to the accommodation 30 for achieving a lever effect. Or else, the plunger component 34 may comprise two push rods that are displaceably mounted laterally at the tube accommodation chamber 1 and that are coupled with the operating handle 16 via a lever or gear mechanism so as to transform a swiveling movement of the operating handle 16 to a pushing movement of the push rods.

The functioning of the tube accommodation chamber 1 and in particular of the tensioning device with a lever mechanism 2 pursuant to the sixth embodiment of the invention is substantially the same as with the fifth embodiment. This means that the swiveling movement of the plate-shaped flap 16 is transformed via the plunger component 34 to a displacing movement of the beam-shaped accommodation 30, wherein, however, simultaneously, for tensioning the tube unit 14 (see middle illustration of Fig. 6) the flap-like operating handle 16 covers the tube accommodation chamber 1 substantially completely when reaching the tensioned final position pursuant to the right illustration in Fig. 6. Also in this case is the tensioning device 2 provided with a self-locking as was already described before by means of the fifth embodiment.

Finally, it is pointed out that the operating handle/operating element need not necessarily be operated manually, but can also be operated by a motor. Such motor may basically be used with all embodiments that have already been described, for instance, so as to apply an operating force on the operating handle/operating element via a gear mechanism or a piston. The use of a motor drive also enables a modification of the tensioning device, as will be described in the following by means of the seventh preferred embodiment pursuant to Fig. 7, middle illustration.

Here, it is basically the matter of a tube accommodation chamber 1 with a stationary pump head 6 and a plate-shaped cover cap 16 that is guided in a displaceable manner at the pump casing 4 or the tube accommodation chamber 1. The cover plate 16 comprises an accommodation for the tube unit 14 consisting of two spaced-apart indentations 12 that are flush with each other and into which the tube unit 14 is adapted to be inserted in an undercut manner by means of holders/engaging elements 22 fixed thereto (see right illustration in Fig. 7). The distance between the two holders 22 is chosen such that the tube 20 in the inserted, but still untensioned condition substantially follows the alignment between the indentations 12.

In the instant case, the drive is formed by an electric motor that is not illustrated and that is operatively connected with the cover plate 16 via a lever mechanism consisting of an eccentric/cam and a plunger component. Alternatively, other constructions for a lever mechanism such as, for instance, a gear mechanism, are, however, also conceivable.

By activating the motor, a pushing force is exerted via the lever mechanism on the cover plate 16, so that it moves from an open final position pursuant to the middle illustration in Fig. 7 in the direction of the pump head 6. In the process of this movement, the tube 20 touches the pump head 6 and is, during the further movement, tensioned around the pump head 6 until the tensioned final position of the cover plate 16. In this case, the self-locking effect is either produced by the motor or the downstream lever mechanism.

In summary, the present invention relates to a peristaltic pump for medical purposes, comprising a pump casing in which a tube accommodation chamber is formed in which a pump head is rotatably mounted, and a tube unit adapted to be inserted in the tube accommodation chamber such that a tube of the tube unit is engaged with the pump head in a manner to encompass same at least partially. For this purpose, a tensioning device is arranged at the pump casing which cooperates with the tube unit so as to tension the tube around the pump head on operation thereof. In accordance with the invention, the tensioning device comprises a lever mechanism, wherein a lever element of the lever mechanism preferably simultaneously forms or comprises a protective cap for the at least partially covering of the tube accommodation.

## Claims

1. A peristaltic pump for medical purposes, comprising a pump casing (4) in which a tube accommodation chamber (1) is formed in which a pump head (6) is rotatably mounted, and a tube unit (14) which is adapted to be inserted or which is inserted in the tube accommodation chamber (1) such that a tube (20) of the tube unit (14) is pulled in longitudinal direction and is engaged with the pump head (6) in a manner to encompass same at least partially, for which purpose a tensioning device (2) is arranged at the pump casing (4) or at the tube accommodation chamber (1), said tensioning device (2) cooperating with the tube unit (14) so as to tension the tube (20) around the pump head (6) on operation thereof, **characterized in that** the tensioning device (2) comprises a lever mechanism.

2. The peristaltic pump according to claim 1, **characterized in that** a lever element (16, 18) of the lever mechanism simultaneously forms or comprises a protective cap for at least partially covering the front side of the tube accommodation chamber (1).

3. The peristaltic pump according to claim 2, **characterized in that** the protective cap either forms or is that lever element that is provided as an operating element or an operating handle (16) of the lever mechanism, or forms or is that lever element (18) that is operable via the operating handle (16) for transferring force.

4. The peristaltic pump according to claim 3, **characterized in that** in case the operating handle (16) simultaneously forms or comprises the protective cap, the protective cap has the shape of a cover plate.

5. The peristaltic pump according to claim 3, **characterized in that** in case the force transferring lever element (18) forms or comprises the protective cap, the operating handle (16) has the shape of a bow-shaped handle, a buckle/operating button, or a toggle lever.

6. The peristaltic pump according to any of the preceding claims, **characterized by** a tube accommodation (12, 30) that is arranged or adapted to be arranged either fixed or displaceable in or at the tube accommodation chamber (1), and into which the tube (20) is adapted to be inserted or is inserted via a tube holder (22) formed or fixed thereon.

7. The peristaltic pump according to claim 6, **characterized in that** in the case of a fixed tube accommodation (12) the lever mechanism acts on the now displaceable pump head (6) so as to press same, by operating the lever mechanism, against a loop-shaped tube middle section (20) formed by the tube accommodation (12) or the tube holder (22), and to extend the tube (20) longitudinally in this process.

8. The peristaltic pump according to claim 6, **characterized in that** in the case of a displaceable tube accommodation (30) the lever mechanism acts on the tube accommodation (30) preferably as a further element of the lever mechanism so as to move same by operation of the lever mechanism relative to the now stationary pump head (6) along with the tube holder (22) such that a loop-shaped tube middle section (20a) formed by the tube accommodation (30) or the tube holder (22) touches the pump head (6) in a manner encompassing same and is extended in longitudinal direction of the tube in this process.

9. The peristaltic pump according to any of the preceding claims, **characterized in that** the lever mechanism is designed to be self-locking.

10. Tube unit of a peristaltic pump in accordance with any one of the preceding claims, with a tube holder (22) holding a flexible, preferably elastic tube (20) at two axially spaced-apart positions and forming at least one undercut adapted to be brought in an undercut holding engagement with a tube accommodation (12, 30) of the peristaltic pump, which tube accommodation (12, 30) cooperates with a lever mechanism of a tensioning device (2) of the peristaltic pump so as to tension the tube (20) on operation of the lever mechanism.

## Patentansprüche

1. Schlauchpumpe zu medizinischen Zwecken mit einem Pumpengehäuse (4), in dem ein Schlauchaufnahmefach (1) ausgebildet ist, in dem ein Pumpenkopf (6) drehbar gelagert ist, sowie mit einer Schlaucheinheit (14), die dazu ausgelegt ist, in das Schlauchaufnahmefach (1) so einsetzbar zu sein oder die so eingesetzt ist, dass ein Schlauch (20) der Schlaucheinheit (14) längsgezogen wird und dabei mit dem Pumpenkopf (6) in diesen zumindest teilweise umspannenden Eingriff kommt, wofür am Pumpengehäuse (4) oder am Schlauchaufnahmefach (1) eine Spannvorrichtung (2) angeordnet ist, die mit der Schlaucheinheit (14) zusammenwirkt, um bei deren Betätigung den Schlauch (20) um den Pumpenkopf (6) zu spannen, **dadurch gekennzeichnet, dass** die Spannvorrichtung (2) einen Hebelmechanismus aufweist.

2. Schlauchpumpe nach Anspruch 1, **dadurch gekennzeichnet, dass** ein Hebelelement (16, 18) des Hebelmechanismus gleichzeitig eine Schutzkappe zur zumindest teilweisen frontseitigen Abdeckung des Schlauchaufnahmefachs (1) bildet oder hat.

3. Schlauchpumpe nach Anspruch 2, **dadurch gekennzeichnet, dass** die Schutzkappe entweder jenes Hebelelement bildet oder ist, das als Betätigungselement oder Handhabe (16) des Hebelmechanismus vorgesehen ist oder jenes Hebelelement (18) bildet oder ist, welches über die Handhabe (16) zur Kraftübertragung betätigbar ist.

4. Schlauchpumpe nach Anspruch 3, **dadurch gekennzeichnet, dass** für den Fall, dass die Handhabe (16) gleichzeitig die Schutzkappe bildet oder hat, diese die Form einer Abdeckplatte aufweist.

5. Schlauchpumpe nach Anspruch 3, **dadurch gekennzeichnet, dass** für den Fall, dass das Kraftübertragungs-Hebelelement (18) die Schutzkappe bildet oder hat, die Handhabe (16) die Form eines Bügelgriffs, einer Schnalle/Betätigungstaste, oder eines Kniehebels aufweist.

6. Schlauchpumpe nach einem der vorstehenden Ansprüche, **gekennzeichnet durch** eine Schlauchaufnahme (12, 30), die entweder fix oder verschiebbar im oder am Schlauchaufnahmefach (1) angeordnet ist oder dazu ausgelegt ist, derart anordenbar zu sein und in die der Schlauch (20) über eine daran ausgebildete oder fixierte Schlauchhalterung (22) einsetzbar oder eingesetzt ist.

7. Schlauchpumpe nach Anspruch 6, **dadurch gekennzeichnet, dass** im Fall einer fixen Schlauchaufnahme (12) der Hebelmechanismus auf den nunmehr verschieblichen Pumpenkopf (6) einwirkt, um diesen durch Betätigung des Hebelmechanismus gegen einen durch die Schlauchaufnahme (12) oder die Schlauchhalterung (22) ausgebildeter, schlaufenförmiger Schlauch-Mittenabschnitt (20a) zu drücken und dabei den Schlauch (20) längs zu dehnen.

8. Schlauchpumpe nach Anspruch 6, **dadurch gekennzeichnet, dass** im Fall einer verschieblichen Schlauchaufnahme (30) der Hebelmechanismus auf die Schlauchaufnahme (30) als weiteres Element des Hebelmechanismus einwirkt, um diese durch Betätigen des Hebelmechanismus relativ zum nunmehr ortsfesten Pumpenkopf (6) zusammen mit der Schlauchhalterung (22) zu bewegen, derart, dass sich ein durch die Schlauchaufnahme (30) oder die Schlauchhalterung (22) ausgebildeter schlaufenförmiger Schlauch-Mittenabschnitt (20a) gegen den Pumpenkopf (6) in diesen umspannender Weise anlegt und dabei in Schlauchlängsrichtung gedehnt wird.

9. Schlauchpumpe nach einem der vorstehenden Ansprüche, **dadurch gekennzeichnet, dass** der Hebelmechanismus selbsthemmend ausgebildet ist.

10. Schlaucheinheit einer Schlauchpumpe gemäß einem der vorstehenden Ansprüche, mit einer Schlauchhalterung (22), welche einen flexiblen, vorzugsweise elastischen Schlauch (20) an zwei axial beabstandeten Positionen festhält und dabei zumindest eine Hinterschneidung ausbildet, die dazu ausgelegt ist, mit einer Schlauchaufnahme (12, 30) der Schlauchpumpe in hinterschneidenden Halte-Eingriff bringbar zu sein, wobei die Schlauchaufnahme (12, 30) mit einem Hebelmechanismus einer Spannvorrichtung (2) der Schlauchpumpe zum Spannen des Schlauchs (20) bei einer Betätigung des Hebelmechanismus zusammenwirkt.

## Revendications

1. Pompe péristaltique à usage médical, comprenant un boîtier de pompe (4) dans laquelle une chambre de logement d'un tube (1) est formée, dans laquelle une tête de pompe (6) est montée en rotation, et une unité de tube (14) qui peut être insérée ou qui est insérée dans la chambre de logement de tube (1), de sorte qu'un tube (20) de l'unité de tube (14) soit tiré dans la direction longitudinale et soit mis en prise avec la tête de pompe (6) de façon à l'englober au moins partiellement, c'est la raison pour laquelle un dispositif de tension (2) est disposé au niveau du boîtier de pompe (4) ou de la chambre de logement de tube (1), ledit dispositif de tension (2) coopérant avec l'unité de tube (14) de façon à tendre le tube (20) autour de la tête de pompe (6) lors de son actionnement, **caractérisée en ce que** le dispositif de tension (2) comprend un mécanisme à levier.

2. Pompe péristaltique selon la revendication 1, **caractérisée en ce qu'**un élément de levier (16, 18) du mécanisme à levier forme simultanément ou comprend un capuchon de protection pour recouvrir au moins partiellement le côté avant de la chambre de logement de tube (1).

3. Pompe péristaltique selon la revendication 2, **caractérisée en ce que** le capuchon de protection forme ou est l'élément de levier qui est fourni comme élément d'actionnement ou comme poignée d'actionnement (16) du mécanisme à levier, ou forme ou est l'élément de levier (18) qui peut être actionné par le biais de la poignée d'actionnement (16) pour transférer une force.

4. Pompe péristaltique selon la revendication 3, **caractérisé en ce que**, si la poignée d'actionnement (16) forme simultanément ou comprend le capuchon de protection, ledit capuchon de protection a la forme d'une plaque de couvercle.

5. Pompe péristaltique selon la revendication 3, **caractérisée en ce que**, si l'élément de levier transférant une force (18) forme ou comprend le capuchon de protection, la poignée d'actionnement (16) a la forme d'une poignée arquée, d'une boucle/un bouton d'actionnement, ou d'un levier basculant.

6. Pompe péristaltique selon l'une quelconque des revendications précédentes, **caractérisée par** un logement de tube (12, 30) disposé ou pouvant être disposé soit de manière fixe ou déplaçable dans ou au niveau de la chambre de logement de tube (1), et dans laquelle le tube (20) peut être inséré ou est inséré par le biais d'un support de tube (22) formé ou fixé dessus.

7. Pompe péristaltique selon la revendication 6, **caractérisée en ce que**, dans le cas d'un logement de tube fixe (12), le mécanisme à levier agit sur la tête de pompe maintenant déplaçable (6) de façon à appuyer sur celle-ci, en actionnant le mécanisme à levier, contre un centre de tube en forme de boucle (20) formé par le logement de tube (12) ou le support de tube (22) et pour étendre le tube (20) longitudinalement dans ce processus.

8. Pompe péristaltique selon la revendication 6, **caractérisée en ce que**, dans le cas d'un logement de tube déplaçable (30), le mécanisme à levier agit sur le logement de tube (30) de préférence comme un élément ultérieur du mécanisme à levier, de façon à le déplacer, par l'actionnement du mécanisme à levier relativement à la tête de pompe maintenant fixe (6) avec le support de tube (22), de sorte qu'une section centrale de tube en forme de boucle (20a) formée par le logement de tube (30) ou le support de tube (22) touche la tête de pompe (6) de façon à l'englober et est étendue dans une direction longitudinale du tube dans ce processus.

9. Pompe péristaltique selon l'une quelconque des revendications précédentes, **caractérisée en ce que** le mécanisme à levier est conçu pour être auto-verrouillable.

10. Unité de tube d'une pompe péristaltique selon l'une quelconque des revendications précédentes, avec un support de tube (22) maintenant un flexible, de préférence un tube élastique (20) dans deux positions axialement espacées et formant au moins un dégagement, pouvant être amené dans une prise du dégagement avec un logement de tube (12, 30) de la pompe péristaltique, ledit logement de tube (12, 30) coopérant avec un mécanisme à levier d'un dispositif de tension (2) de la pompe péristaltique, de façon à tendre le tube (20) lors de l'actionnement du mécanisme à levier.
